# EUROPÄISCHE PATENTANMELDUNG

(11) **EP 4 595 741 A1**
(43) Veröffentlichungstag der Anmeldung: **06.08.2025**
(21) Anmeldenummer: 25155078.6
(22) Anmeldetag: 30.01.2025
(51) Int. Cl.: A01D 65/00, G01N 21/84

(54) **AGRARVORRICHTUNG, VERFAHREN SOWIE COMPUTERIMPLEMENTIERTES VERFAHREN ZUM ERFASSEN UND/ODER ZUM ANALYSIEREN WENIGSTENS EINER PFLANZE**

(30) Priorität: 30.01.2024 DE 102024102567
(71) Anmelder: Digital workbench gmbh, 85139 Wettstetten (DE)
(72) Erfinder: Schmidt, Josef, 85139 Wettstetten (DE)
(74) Vertreter: Canzler & Bergmeier Patentanwälte Partnerschaft mbB

(57) **Zusammenfassung**

Die Erfindung betrifft eine Agrarvorrichtung (1) zum Erfassen und/oder Analysieren wenigstens einer Pflanze (2), insbesondere Getreidepflanze, mit wenigstens einer Positioniereinheit (3) zum Positionieren, Auslenken, Auffächern und/oder Separieren der wenigstens einen Pflanze (2) und/oder wenigstens einer aus mehreren aneinandergereihten Pflanzen (2) ausgebildeten Pflanzenreihe (4), und mit wenigstens einer Analyseeinheit (5) zum Erfassen und/oder Analysieren der wenigstens einen Pflanze (2). Ferner betrifft die Erfindung eine Vereinzelungseinrichtung (17), ein Verfahren sowie ein computerimplementiertes Verfahren zum Erfassen und/oder zum Analysieren wenigstens einer Pflanze (2).

## Beschreibung

Die vorliegende Erfindung betrifft eine Agrarvorrichtung, insbesondere eine Boniturvorrichtung, zum Erfassen und/oder Analysieren wenigstens einer Pflanze, insbesondere Getreidepflanze, mit wenigstens einer Positioniereinheit zum Positionieren, Auslenken, Auffächern und/oder Separieren der wenigstens einen Pflanze und/oder wenigstens einer aus mehreren aneinandergereihten Pflanzen ausgebildeten Pflanzenreihe, und mit wenigstens einer Analyseeinheit zum Erfassen und/oder Analysieren der wenigstens einen Pflanze. Ferner betrifft die Erfindung eine Vereinzelungseinrichtung, ein Verfahren sowie ein computerimplementiertes Verfahren zum Erfassen und/oder zum Analysieren wenigstens einer Pflanze.

Die Analyse bzw. die Bonitur von Pflanzen, insbesondere von Getreidepflanzen, erfolgt in der Agrarwirtschaft nach wie vor manuell durch Personen. Die Anzahl der infrage kommenden Personen schrumpf jedoch. Zusätzlich oder alternativ ist die Beschäftigung der Personen zeit- und kostenintensiv. Deshalb wurde untersucht, inwieweit die Erfassung und/oder Analyse der Pflanzen mit Hilfe von Drohnen aus der Luft und meist aus größerer Entfernung erfolgen kann. Es hat sich jedoch gezeigt, dass diese Erkennung und/oder Analyse zu ungenau und fehleranfällig ist.

Aufgabe der vorliegenden Erfindung ist es, die aus dem Stand der Technik bekannten Nachteile zu beseitigen. Aufgabe ist es insbesondere eine Agrarvorrichtung, ein Verfahren und/oder ein computerimplementiertes Verfahren zu schaffen, mit dessen Hilfe das Erfassen und/oder Analysieren der Pflanze vereinfacht, verbessert und/oder automatisiert erfolgt.

Die Aufgabe wird gelöst durch eine Agrarvorrichtung, ein Verfahren und/oder ein computerimplementiertes Verfahren mit den Merkmalen der unabhängigen Patentansprüche oder des unabhängigen Patentanspruchs. Vorteilhafte oder bevorzugte Ausführungen oder Weiterbildungen der Erfindung sind durch die Merkmale der abhängigen Ansprüche gekennzeichnet.

Vorgeschlagen wird eine Agrarvorrichtung, insbesondere eine Boniturvorrichtung, zum Erfassen und/oder Analysieren wenigstens einer Pflanze, insbesondere Getreidepflanze. Als Bonitur ist die fachgerechte, qualitative Beurteilung landwirtschaftlicher Betrachtungsobjekte zu verstehen. Beim Bonieren können beispielsweise Pflanzenmerkmale erhoben werden. So werden häufig Blattlängen gemessen, Insekten gezählt, Schädlinge detektiert und/oder Krankheiten erkannt.

Die Agrarvorrichtung umfasst vorzugsweise wenigstens eine Positioniereinheit zum Positionieren, Auslenken, Auffächern und/oder Separieren der wenigstens einen Pflanze und/oder wenigstens einer aus mehreren aneinandergereihten Pflanzen ausgebildeten Pflanzenreihe. Des Weiteren umfasst die Agrarvorrichtung vorzugsweise wenigstens eine Analyseeinheit zum Erfassen und/oder Analysieren der wenigstens einen Pflanze.

Vorteilhaft ist es, wenn die Positioniereinheit wenigstens ein Leitelement zum Führen, Umlenken und/oder Auslenken der wenigstens einen Pflanze und/oder der wenigstens einen Pflanzenreihe und/oder wenigstens ein, insbesondere stangenförmiges, Aufnahmeelement für das wenigstens eine Leitelement umfasst.

Vorteilhaft ist es zudem, wenn das wenigstens eine Leitelement an dem wenigstens einen Aufnahmeelement angeordnet ist und/oder entlang einer Längsrichtung und/oder Querrichtung der Agrarvorrichtung von dem wenigstens einen Aufnahmeelement absteht.

Auch ist es von Vorteil, wenn das wenigstens eine Leitelement zumindest abschnittsweise schräg zu der Längsrichtung und/oder der Querrichtung verläuft und/oder wenigstens eine Biegung umfasst, wobei das wenigstens eine Leitelement vorzugsweise mit der wenigstens einen Biegung, insbesondere mit zwei Biegungen, in einer Draufsicht der Agrarvorrichtung S-förmig ausgebildet ist.

Vorteilhaft ist es zudem, wenn das wenigstens eine Leitelement einen, insbesondere als Führungssteg und/oder Führungsbügel ausgebildeten, Führungsabschnitt umfasst, mit dessen Hilfe die wenigstens eine Pflanze, insbesondere im Bereich eines Halms der wenigstens einen Pflanze, im bestimmungsgemäßen Gebrauch der Agrarvorrichtung geführt und/oder ausgelenkt werden kann.

Des Weiteren ist es vorteilhaft, wenn das wenigstens eine Leitelement einen Umlenkabschnitt umfasst, mit dessen Hilfe die wenigstens eine Pflanze, insbesondere im Bereich wenigstens einer Frucht, beispielsweise ein Korn und/oder eine Ähre, und/oder eines Blatts der wenigstens einen Pflanze, im bestimmungsgemäßen Gebrauch der Agrarvorrichtung geführt und/oder ausgelenkt werden kann.

Auch ist es von Vorteil, wenn sich der Führungsabschnitt zumindest abschnittsweise entlang der Längsrichtung erstreckt und/oder der Umlenkabschnitt zu der Querrichtung und/oder der Längsrichtung schräg verläuft und/oder die Biegung umfasst, so dass die wenigstens eine Pflanze mittels des Führungsabschnitts zum Umlenkabschnitt geführt und/oder mittels des Umlenkabschnitts entlang der Querrichtung ausgelenkt wird.

Ebenso ist es vorteilhaft, wenn das wenigstens eine Leitelement, insbesondere im Bereich des wenigstens einen Umlenkabschnitts, wenigstens eine Stützstruktur umfasst, wobei das wenigstens eine Leitelement mittels der Stützstruktur an dem wenigstens einen Aufnahmeelement angeordnet und/oder befestigt ist und/oder den Umlenkabschnitt aufspannt.

Auch ist es vorteilhaft, wenn das wenigstens eine Leitelement, insbesondere der Umlenkabschnitt des Leitelements, entlang einer Hochrichtung der Agrarvorrichtung gebogen und/oder gekrümmt ausgebildet ist, so dass sich die durch den Umlenkabschnitt entlang der Querrichtung eingebrachte Auslenkung der Pflanze entlang der Hochrichtung ändert, insbesondere in Hochrichtung zunimmt.

Vorteilhaft ist es zudem, wenn der wenigstens eine Führungsabschnitt und der wenigstens eine Umlenkabschnitt aneinander angeordnet sind und/oder tangential ineinander übergehen.

Auch ist es von Vorteil, wenn die wenigstens eine Analyseeinheit wenigstens einen Bildsensor zum Erfassen eines Ist-Zustands der wenigstens einen Pflanze, beispielsweise der Biomasse, Ertragskennzahl, Krankheiten, Schädlingsbefall und/oder Morphologie, umfasst, wobei der Bildsensor vorzugsweise als RGB-Bildsensor, NIR-Bildsensor, SWIR-Bildsensor, MWIR-Bildsensor, LWIR-Bildsensor, MSI-Bildsensor und/oder HSI-Bildsensor ausgebildet ist.

Als RGB-Bildsensor ist ein Bildsensor zu verstehen, der beispielsweise Licht in den Farben Rot, Grün und Blau zur Erstellung von Farbbildern erfasst. Der RGB-Bildsensor könnte beispielsweise grundlegende visuelle Merkmale von Pflanzen erkennen. Als NIR-Bildsensor ist ein Bildsensor zu verstehen, der beispielsweise Licht im nahen Infrarotbereich (700-1000 nm) erfasst, um vorzugsweise unsichtbare Details sichtbar zu machen. Der NIR-Bildsensor könnte beispielsweise die Vitalität und/oder den Wassergehalt von Pflanzen analysieren. Als SWIR-Bildsensor ist ein Bildsensor zu verstehen, der beispielsweise Licht im kurzwelligen Infrarotbereich erfasst, um vorzugsweise Materialien und/oder Oberflächen zu analysieren. Der SWIR-Bildsensor könnte beispielsweise Materialunterschiede und/oder spezifische Pflanzeneigenschaften sichtbar machen. Als MWIR-Bildsensor und LWIR-Bildsensor sind Bildsensoren zu verstehen, der beispielsweise Licht im mittleren und langen Wellenbereich erfassen. Als MSI-Bildsensor ist ein Bildsensor zu verstehen, der beispielsweise Bilder in mehreren spektralen Bändern aufnimmt. Der MSI-Bildsensor könnte beispielsweise spezifische Eigenschaften wie Chlorophyllgehalt und/oder Blattgesundheit analysieren. Als HSI-Bildsensor ist ein Bildsensor zu verstehen, der beispielsweise kontinuierliche Spektralinformationen für jede Pixelposition zur detaillierten Materialanalyse erfasst. Der HSI-Bildsensor könnte beispielsweise detaillierte Informationen über die chemische Zusammensetzung und/oder den Zustand von Pflanzen erfassen.

Zusätzlich oder alternativ ist der Bildsensor vorzugsweise als UV-Bildsensor, TIR-Bildsensor, ToF-Bildsensor und/oder polarimetrischer Sensor ausgebildet. Als UV-Bildsensor ist ein Bildsensor zu verstehen, der beispielsweise Licht im ultravioletten Spektralbereich erfasst, um Oberflächenbeschaffenheiten oder biologische Materialien sichtbar zu machen. So könnten mittels des UV-Bildsensors beispielsweise Oberflächenschäden, Wachse und/oder Pigmente von Pflanzen sichtbar gemacht werden. Als TIR-Bildsensor ist ein Bildsensor zu verstehen, der beispielsweise langwelliges Infrarotlicht erfasst, um Temperaturverteilungen darzustellen. So könnte mittels des TIR-Bildsensors der Wasserstress und/oder die Transpiration von Pflanzen gemessen werden. Als ToF-Bildsensor ist ein Bildsensor zu verstehen, der beispielsweise die Entfernung zu Pflanzen misst, um vorzugsweise 3D-Strukturen und/oder Pflanzenhöhen zu analysieren. Als polarimetrischer Sensor ist ein Sensor zu verstehen, der die Polarisationseigenschaften des reflektierten Lichts erfasst. Dieser kann somit zusätzliche Informationen über die Oberfläche und Struktur der Pflanze liefern. Zusätzlich oder alternativ ist der Bildsensor vorzugsweise als Fluoreszenz-Bildsensor, Lidar-Sensor, Raman-Spektroskopie-Sensor, Hyperspektraler Fluoreszenz-Sensor und/oder Multispektraler Polarimetrischer Sensor ausgebildet. Zusätzlich oder alternativ umfasst die wenigstens eine Analyseeinheit mehrere der Bildsensoren.

Zusätzlich oder alternativ umfasst die wenigstens eine Analyseeinheit wenigstens einen Sensor, insbesondere einen Spektroradiometer, einen Chlorophyll-Meter, einen Elektrochemische Sensor, einen Gassensoren (CO₂, O₂, Ethylen), einen Drucksensor, einen akustischen Sensor, einen Biosensor, einen Mikrowellensensor und/oder einen Radarsensor, umfasst.

Des Weiteren ist es vorteilhaft, wenn dem wenigstens einen Leitelement die wenigstens eine Analyseeinheit zugeordnet ist, wobei vorzugsweise das wenigstens eine Leitelement und die wenigstens eine zugeordnete Analyseeinheit, insbesondere entlang einer Erfassungsachse des Bildsensors der Analyseeinheit, voneinander beabstandet sind.

Vorteile bringt es zudem mit sich, wenn das wenigstens eine Leitelement, insbesondere der Umlenkabschnitt des Leitelements, einen, insbesondere geschlossenen, homogenen, einfarbigen und/oder einseitig am Leitelement angeordneten, Analyseabschnitt umfasst, wobei die Erfassungsachse des Bildsensors im Bereich des Analyseabschnitts auf das wenigstens eine Leitelement auftrifft und/oder die wenigstens eine Pflanze, vorzugsweise die Frucht und/oder das Blatt der wenigstens einen Pflanze, im bestimmungsgemäßen Gebrauch der Agrarvorrichtung entlang der Erfassungsachse zwischen dem Analyseabschnitt des Leitelements und dem Bildsensor der Analyseeinheit angeordnet ist.

Vorteile bringt es mit sich, wenn die Agrarvorrichtung wenigstens eine Abschirmeinrichtung zum Abschirmen der Pflanzen vor natürlichem Licht und/oder wenigstens eine Lichtquelle zum Erzeugen von künstlichem Licht umfasst, wobei die wenigstens eine Lichtquelle derart ausgebildet ist, dass wenigstens eine der Pflanzen und/oder der Analyseabschnitt zumindest zeitweise beleuchtet wird. Vorzugsweise ist die Lichtquelle als Blitzlicht ausgebildet und/oder angesteuert, so dass die Pflanze und/oder der Analyseabschnitt kurzzeitig mit hoher Leuchtintensität beleuchtet werden kann. Die Abschirmeinrichtung kann beispielsweise als Plane und/oder Schirm ausgebildet und/oder oberhalb der Positioniereinheit angeordnet sein.

Ebenso bringt es Vorteile mit sich, wenn die Agrarvorrichtung wenigstens eine Vereinzelungseinrichtung zum Separieren, Aufreihen, Trennen, Stabilisieren und/oder Begrenzen der Pflanzenreihe in einzelne Pflanzen umfasst, wobei die Vereinzelungseinrichtung vorzugsweise wenigstens ein Trennelement und/oder ein Förderband und/oder einen Riemen und/oder eine Riemenbürste und/oder einen Zahnriemen und/oder ein Förderrad und/oder ein Andrückelement und/oder ein Federelement und/oder eine Düse aufweist. Beim Begrenzen wird vorzugsweise die Bewegung bzw. die Auslenkung begrenzt. Das Trennelement kann beispielsweise als Bürste, Zahn, Stab, Bolzen und/oder Finger ausgebildet sein. Das wenigstens eine Trennelement, insbesondere viele Trennelemente, kann beispielsweise an dem Riemen angeordnet sein. Der Riemen kann sich zum Vereinzeln der Pflanzen entlang seiner Umfangsrichtung bewegen und so einzelne Pflanzen aufnehmen. Mit Hilfe des Andrückelement und/oder dem Federelement und/oder der Düse kann die Pflanze nach dem Vereinzeln stabilisiert und/oder in seiner Bewegung begrenzt werden. So kann die Analyse verbessert werden. Zusätzlich oder alternativ kann die Düse die Pflanzen vereinzeln. Die Düse kann einen Luftstrom und/oder einen Druckluftstrom ausgeben.

Zusätzlich oder alternativ umfasst die Vereinzelungseinrichtung vorzugsweise wenigstens ein Förderelement und/oder eine Spindel und/oder eine Spirale und/oder eine Schneckenwelle. Durch die Verwendung dieser Elemente kann eine präzise und/oder schonende Vereinzelung der Pflanzen erreicht werden. Das Förderband und/oder der Riemen und/oder die Riemenbürste und/oder der Zahnriemen und/oder das Förderrad und/oder eine Spindel und/oder eine Spirale und/oder eine Schneckenwelle kann/können beispielsweise als Vereinzelungselement€ bezeichnet werden. Zusätzlich oder alternativ kann das Förderband und/oder der Riemen und/oder die Riemenbürste und/oder der Zahnriemen und/oder das Förderrad als Andrückelement verwendet werden, so dass die Pflanze in dem Förderelement und/oder dem Förderband und/oder dem Riemen und/oder der Riemenbürste und/oder dem Zahnriemen und/oder der Schneckenwelle und/oder der Spindel und/oder der Spirale und/oder dem Förderrad gehalten wird.

Vorteilhaft ist es zudem, wenn das wenigstens eine Förderelement und/oder das Förderband und/oder der Riemen und/oder die Riemenbürste und/oder der Zahnriemen und/oder die wenigstens eine Schneckenwelle und/oder die wenigstens eine Spindel und/oder die wenigstens eine Spirale und/oder das wenigstens eine Förderrad, das wenigstens Trennelement und/oder den wenigstens einen Trennabschnitt umfasst. Mittels des Trennelements und/oder des Trennabschnitts können wenigstens zwei Pflanzen voneinander getrennt werden. Zwei Pflanzen der Pflanzenreihe sind im bestimmungsgemäßen Gebrauch der Vereinzelungseinrichtung, vorzugsweise an unterschiedlichen Seiten des Trennelements und/oder des Trennabschnitts, angeordnet. Dies trägt dazu bei, die Erkennung zu verbessern, da unerwünschte Überlappungen und/oder Verwicklungen der Pflanzen reduziert werden.

Bei der Schneckenwelle und/oder der Spindel und/oder der Spirale können einzelne Wendelabschnitte als Trennelement und/oder Trennabschnitte bezeichnet werden. Bei der Schneckenwelle und/oder der Spindel und/oder der Spirale können mehrere der Trennelemente und/oder Trennabschnitte somit miteinander verbunden sein, so dass die Wendel und/oder mehrere Wendeln (bei Ganganzahl größer 1) aus den mehreren Trennelementen und/oder Trennabschnitten gebildet ist.

Auch ist es vorteilhaft, wenn sich das wenigstens eine Förderelement und/oder das Förderband und/oder der Riemen und/oder die Riemenbürste und/oder der Zahnriemen und/oder die Schneckenwelle und/oder die Spindel und/oder die Spirale und/oder das Förderrad entlang der Längsrichtung und/oder entlang einer Hochrichtung und/oder entlang einer Drehachse erstreckt. Zusätzlich oder alternativ umfasst die Schneckenwelle und/oder die Spindel und/oder die Spirale die wenigstens eine Drehachse. Die Drehachse ist dabei die Achse, um die sich die Schneckenwelle und/oder die Spindel und/oder die Spirale dreht und/oder drehbar gelagert ist. Vorzugsweise erstreckt sich die Drehachse entlang der Längsrichtung und/oder entlang der Hochrichtung. Zusätzlich oder alternativ verläuft die Drehachse parallel zu einer aus der Längsrichtung und der Hochrichtung aufgespannten Ebene. Zusätzlich oder alternativ wird das wenigstens eine Förderelement und/oder das Förderband und/oder der Riemen und/oder die Riemenbürste und/oder der Zahnriemen und/oder die Schneckenwelle und/oder die Spindel und/oder die Spirale und/oder das Förderrad entlang der Längsrichtung (gemeinsam mit der Agrarvorrichtung) entlang der Pflanzenreihe bewegt.

Des Weiteren ist es von Vorteil, wenn das wenigstens eine Förderelement und/oder das Förderband und/oder der Riemen und/oder die Riemenbürste und/oder der Zahnriemen und/oder die wenigstens eine Schneckenwelle und/oder die wenigstens eine Spindel und/oder die wenigstens eine Spirale und/oder das wenigstens eine Förderrad mehrere entlang der Längsrichtung und/oder entlang der Hochrichtung der Agrarvorrichtung und/oder entlang der Drehachse voneinander beabstandete Trennelemente und/oder Trennabschnitte umfasst. Dadurch können die Pflanzen voneinander getrennt und/oder zwischen den Trennelementen und/oder den Trennabschnitten angeordnet werden.

Zudem ist es vorteilhaft, wenn sich ein Abstand zwischen den mehreren Trennelementen und/oder Trennabschnitte entlang der Längsrichtung und/oder der Hochrichtung und/oder entlang einer Drehachse zumindest abschnittsweise verändert. Zusätzlich oder alternativ ist es vorteilhaft, wenn sich eine Wandstärke der mehreren Trennelemente und/oder Trennabschnitte entlang der Längsrichtung und/oder der Hochrichtung und/oder entlang einer Drehachse zumindest abschnittsweise verändert. Die Drehachse ist vorzugsweise die Drehachse der Schneckenwelle, der Spindel und/oder der Spirale.

Vorzugsweise bewegt sich die Agrarvorrichtung entlang und/oder entgegen der Längsrichtung. Auch die Pflanzenreihen erstrecken sich vorzugsweise entlang der Längsrichtung. Die einzelnen Pflanzen erstrecken sich entlang der Hochrichtung bzw. sind entlang der Hochrichtung gewachsen. So ist im Bereich eines Untergrunds eine Wurzel der Pflanze angeordnet. Die Frucht, welche vorzugsweise erfasst werden soll, ist über einen Halm von der Wurzel beabstandet. Die sich entlang der Längsrichtung und/oder der Hochrichtung und/oder der Drehachse zumindest abschnittsweise verändernden Abstände und/oder Wandstärken können die Pflanzen auffächern und/oder vereinzeln.

So verändern sich die Abstände und/oder die Wandstärken entlang der Längsrichtung bzw. entlang der Bewegungsrichtung der Agrarvorrichtung so, dass die Pflanzen bei kleinen Abständen und/oder Wandstärken aufgenommen werden. Bei der weiteren Bewegung der Agrarvorrichtung werden durch die größer werdenden Abstände und/oder Wandstärken die Pflanzen aufgefächert und/oder vereinzelt. Die sich verändernden Abstände und/oder Wandstärken ermöglichen eine schrittweise und kontrollierte Vereinzelung. Dies führt zu einer hohen Effizienz und reduziert die Gefahr, dass Pflanzen beim Vereinzeln beschädigt werden. Durch diese Bewegung in Verbindung mit dem veränderlichen Abstand und/oder der Wandstärke können die Pflanzen, insbesondere im Bereich der Frucht, voneinander separiert werden.

Vorteilhaft ist es zudem, wenn die wenigstens eine Schneckenwelle, die wenigstens eine Spindel und/oder die wenigstens eine Spirale wenigstens eine Wendel umfasst. Vorzugsweise umfasst die wenigstens eine Schneckenwelle, die wenigstens eine Spindel und/oder die wenigstens eine Spirale wenigstens zwei Wendeln. So kann eine größere Steigung der Wendel resultieren, wodurch das Auffächern und/oder Vereinzeln weiter verbessert und/oder gesteigert werden kann.

Zusätzlich oder alternativ verjüngt sich die wenigstens eine Schneckenwelle, die wenigstens eine Spindel und/oder die wenigstens eine Spirale, insbesondere konisch, zu einem Anfangsabschnitt. Der Anfangsabschnitt ist zu dem Untergrund und/oder zu der wenigstens einen Pflanze ausgerichtet. Insbesondere kommt die wenigstens eine Schneckenwelle, die wenigstens eine Spindel und/oder die wenigstens eine Spirale mit dem Anfangsabschnitt als erstes mit der Pflanze in Berührung. So kann die wenigstens eine Schneckenwelle, die wenigstens eine Spindel und/oder die wenigstens eine Spirale vereinfacht zwischen den Pflanzenreihen eintauchen. Zusätzlich oder alternativ können die Trennelemente und/oder die Trennabschnitte und/oder Wendelabschnitte im Bereich des Anfangsabschnitt den geringsten Abstand, die geringste Wandstärke und/oder die geringste Wendelhöhe umfassen. Dadurch ist ein möglichst sanftes und/oder sicheres Aufgreifen der Pflanzen, insbesondere an deren Wurzel, Wurzelansatz und/oder im unteren Drittel des Halms, möglich. Vorzugsweise stellt die Wendel das wenigstens eine Trennelement und/oder den wenigstens einen Trennabschnitt bereit.

Zudem ist es vorteilhaft, wenn die Vereinzelungseinrichtung wenigstens ein Antriebselement zum Antreiben des Förderelements und/oder des Förderbands und/oder des Riemens und/oder der Riemenbürste und/oder des Zahnriemens und/oder der Schneckenwelle und/oder der Spindel und/oder der Spirale und/oder des Förderrads und/oder wenigstens eine Oszillationseinrichtung zum Einbringen einer oszillierenden Bewegung in die Pflanze umfasst.

Des Weiteren ist es vorteilhaft, wenn die wenigstens eine Vereinzelungseinrichtung dem wenigstens einen Leitelement, insbesondere dem Umlenkabschnitt und/oder dem Analyseabschnitt des wenigstens einen Leitelements, zugeordnet ist, so dass die wenigstens eine durch das wenigstens eine Leitelement umgelenkte Pflanze der Pflanzenreihe zum Erfassen und/oder Analysieren aneinander angereiht, voneinander getrennt und/oder an den Analyseabschnitt angedrückt wird.

Vorteilhaft ist es zudem, wenn die Positioniereinheit wenigstens eine Stelleinrichtung umfasst, wobei vorzugsweise eine erste Stelleinrichtung als Linearstelleinrichtung zum Verstellen des wenigstens einen Leitelements entlang der Hochrichtung und/oder Längsrichtung, so dass beispielsweise Pflanzen, Früchte und/oder Blätter unterschiedlicher Höhen erfassbar und/oder analysierbar sind, und/oder eine zweite Stelleinrichtung als Rotationsstelleinrichtung zum Verdrehen des wenigstens einen Leitelements und/oder als Linearstelleinrichtung zum Verstellen des wenigstens einen Leitelements entlang der Querrichtung ausgebildet ist, so dass beispielsweise Pflanzen, Früchte und/oder Blätter von Pflanzenreihen unterschiedlicher Breiten erfassbar und/oder analysierbar sind. Zusätzlich oder alternativ kann die erste Stelleinrichtung und/oder die zweite Stelleinrichtung abhängig von der wenigstens einen Längsverstellung und/oder Querverstellung und/oder Höhenverstellung der Bewegungseinheit verstellt werden.

Vorteile bringt es zudem mit sich, wenn das wenigstens eine Aufnahmeelement die erste Stelleinrichtung umfasst und/oder an der zweiten Stelleinrichtung angeordnet ist, wobei das Aufnahmeelement umfassend die erste Stelleinrichtung vorzugsweise als Teleskopstange ausgebildet ist.

Ebenso bringt es Vorteile mit sich, wenn die Agrarvorrichtung wenigstens eine Behandlungseinheit zum Düngen und/oder Behandeln und/oder wenigstens eine Markierungseinheit zum Markieren der wenigstens einen Pflanze umfasst.

Vorteilhaft ist es, wenn die Agrarvorrichtung mehrere Leitelemente und mehrere Analyseeinheiten umfasst, wobei wenigstens einem der Leitelemente und/oder mehreren der Leitelemente wenigstens eine der Analyseeinheiten zugeordnet ist. So ist es vorstellbar, dass mehrere Leitelemente die wenigstens eine Pflanzenreihe und/oder mehrere Pflanzenreihen derart führen, umlenken und/oder auslenken, dass zwischen zwei Leitelementen eine der Pflanzenreihe, insbesondere als freistehende Pflanzenreihe, angeordnet ist.

Diese freistehende Pflanzenreihe kann im Anschluss mit Hilfe der wenigstens einen Analyseeinheit und/oder mehreren Analyseeinheiten erfasst und/oder analysiert werden. So ist es denkbar, dass die eine oder die mehreren Analyseeinheiten den einen oder mehrere der Bildsensoren und/oder Sensoren umfassen. So ist eine mehrdimensionale Erfassung und/oder Analyse realisierbar.

Vorteilhaft ist es zudem, wenn die dem wenigstens einen Leitelement zugeordnete Analyseeinheit und/oder Vereinzelungseinrichtung an einem nebengeordneten Leitelement der Positioniereinheit, insbesondere an der Stützstruktur des nebengeordneten Leitelements, angeordnet ist, so dass beim Verstellen des wenigstens einen Leitelements und/oder der Positioniereinheit mittels der wenigstens einen Stelleinrichtung die wenigstens eine Analyseeinheit und/oder Vereinzelungseinrichtung mitverstellt wird.

Ebenso ist es vorteilhaft, wenn die Agrarvorrichtung wenigstens eine Bewegungseinheit zum Bewegen der Agrarvorrichtung entlang der Längsrichtung der Agrarvorrichtung, entlang der wenigstens einen Pflanze und/oder entlang der wenigstens einen entlang der Längsrichtung erstreckenden Pflanzenreihe umfasst. Zusätzlich oder alternativ umfasst die Agrarvorrichtung, insbesondere die Bewegungseinheit wenigstens eine Längsverstellung und/oder eine Querverstellung und/oder eine Höhenverstellung, mit deren Hilfe die Bewegungseinheit, beispielsweise wenigstens ein Rad und/oder ein Fahrwerk der Bewegungseinheit, verstellbar sind.

Ebenso bringt es Vorteile mit sich, wenn die Agrarvorrichtung als autonomer mobiler, insbesondere fahrender und/oder fliegender, Roboter ausgebildet ist.

Zusätzlich oder alternativ ist es vorteilhaft, wenn die Agrarvorrichtung als stationäre Agrarvorrichtung ausgebildet ist. Zusätzlich oder alternativ ist die wenigstens eine Positioniereinheit und/oder das wenigstens eine Leitelement bewegbar ausgebildet. Die bewegbare Ausgestaltung kann beispielsweise mit Hilfe der wenigstens einen Stelleinrichtung, insbesondere mit Hilfe der wenigstens einen ersten Stelleinrichtung und/oder der wenigstens einen zweiten Stelleinrichtung, gewährleistet werden.

Zusätzlich oder alternativ ist die Agrarvorrichtung als bewegbare Agrarvorrichtung ausgebildet. Hierbei kann die Agrarvorrichtung beispielsweise die bereits erwähnte Bewegungseinheit umfassen. Die Agrarvorrichtung, insbesondere die bewegbare Agrarvorrichtung kann beispielsweise fremdbewegt und/oder eigenbewegt werden. Die bewegbare Agrarvorrichtung kann beispielsweise durch einen Anwender und/oder eine zweite Agrarvorrichtung und/oder eine Landmaschine und/oder einem Zugfahrzeug, insbesondere Traktor, bewegt, insbesondere geschoben und/oder gezogen, werden. Zusätzlich oder alternativ umfasst die Agrarvorrichtung wenigstens einen Antrieb. Die bewegbare Agrarvorrichtung, insbesondere die Bewegungseinheit, umfasst vorzugsweise wenigstens ein Rad und/oder wenigstens eine Tragfläche und/oder wenigstens einen Flügel und/oder wenigstens ein Rotorblatt. Zusätzlich oder alternativ ist die Agrarvorrichtung mit Hilfe wenigstens eines Führungselementes, insbesondere wenigstens eines Fahrwegs und/oder wenigstens einer Schiene, geführt.

Vorteile bringt es zudem mit sich, wenn die Positioniereinheit wenigstens einen Befestigungsabschnitt umfasst, wobei mittels des Befestigungsabschnitts die Positioniereinheit, die Analyseeinheit, die Behandlungseinheit und/oder die Vereinzelungseinrichtung, insbesondere verschiebefest, an der Bewegungseinheit der Agrarvorrichtung angeordnet ist.

Ebenso bringt es Vorteile mit sich, wenn die Agrarvorrichtung wenigstens eine Sensoreinheit zum Erfassen von Umgebungsdaten, eine Funkeinheit zum Empfangen und/oder Versenden von Umgebungsdaten und/oder eine Ortungseinheit zur Ortung der aktuellen Position der Agrarvorrichtung umfasst.

Ebenso ist es vorteilhaft, wenn die Agrarvorrichtung wenigstens eine Recheneinheit zum Steuern und/oder Regeln der Agrarvorrichtung, insbesondere der Positioniereinheit, der Analyseeinheit, der Behandlungseinheit, der Vereinzelungseinrichtung, der Bewegungseinheit, der Sensoreinheit, der Funkeinheit und/oder der Ortungseinheit und/oder des Antriebselements und/oder der Oszillationseinrichtung umfasst. So ist es beispielsweise möglich das wenigstens eine Antriebselement abhängig von der Bewegungseinheit zu steuern. Die Bewegung, insbesondere Drehbewegung, des Förderelements und/oder des Förderbands und/oder des Riemens und/oder der Riemenbürste und/oder des Zahnriemens und/oder der Schneckenwelle und/oder der Spindel und/oder der Spirale und/oder des Förderrads wird vorzugsweise in Abhängigkeit der Bewegung, insbesondere Linearbewegung entlang und/oder entgegen der Längsrichtung, der Agrarvorrichtung gesteuert und/oder angepasst. Wird die Agrarvorrichtung entlang und/oder entgegen der Längsrichtung schneller bewegt, so wird auch das Förderelement und/oder das Förderband und/oder der Riemen und/oder die Riemenbürste und/oder der Zahnriemen und/oder die Schneckenwelle und/oder die Spindel und/oder die Spirale und/oder das Förderrad schneller bewegt, insbesondere schneller um die Drehachse gedreht.

Vorteile bringt es mit sich, wenn die Agrarvorrichtung wenigstens eine Speichereinheit zum Speichern des durch die Analyseeinheit erfassten Ist-Zustands und/oder eines Soll-Zustands der Pflanze, beispielsweise die Biomasse, Ertragskennzahl, Krankheiten, Schädlingsbefall und/oder Morphologie, umfasst.

Auch ist es von Vorteil, wenn die Recheneinheit ein Computerprogramm umfasst, wobei das Computerprogramm vorzugsweise eine künstliche Intelligenz aufweist und/oder mittels maschinellem Lernen weiterentwickelt wird, so dass der durch die Analyseeinheit erfasste Ist-Zustand der Pflanze, beispielsweise die Biomasse, Ertragskennzahl, Krankheiten, Schädlingsbefall und/oder Morphologie, analysiert und/oder kategorisiert werden kann, insbesondere um den Ist-Zustand der wenigstens einen Pflanze einen mit dem Ist-Zustand wenigstens einer anderen Pflanze und/oder einem Soll-Zustand zu vergleichen und/oder um die wenigstens eine Pflanze zu behandeln, zu markieren und/oder zu düngen.

Des Weiteren wird eine Vereinzelungseinrichtung für eine Agrarvorrichtung vorgeschlagen. Die Agrarvorrichtung ist vorzugsweise gemäß der vorherigen und/oder nachfolgenden Beschreibung ausgebildet, wobei die genannten Merkmale einzeln oder in beliebiger Kombination vorhanden sein können.

Die Vereinzelungseinrichtung umfasst wenigstens ein in der vorherigen und/oder nachfolgenden Beschreibung genanntes und die Vereinzelungseinrichtung betreffendes Merkmal. Die genannten Merkmale der Vereinzelungseinrichtung können einzeln oder in beliebiger Kombination vorhanden sein.

Ferner wird eine Positioniereinheit für eine Agrarvorrichtung vorgeschlagen. Die Agrarvorrichtung ist vorzugsweise gemäß der vorherigen und/oder nachfolgenden Beschreibung ausgebildet, wobei die genannten Merkmale einzeln oder in beliebiger Kombination vorhanden sein können.

Die Positioniereinheit umfasst wenigstens ein in der vorherigen und/oder nachfolgenden Beschreibung genanntes und die Positioniereinheit betreffendes Merkmal. Die genannten Merkmale der Positioniereinheit können einzeln oder in beliebiger Kombination vorhanden sein.

Vorgeschlagen wird ferner ein Verfahren zum Erfassen und/oder Analysieren wenigstens einer Pflanze, insbesondere Getreidepflanze. Das Verfahren wird vorzugsweise mittels einer Agrarvorrichtung, insbesondere gemäß der vorherigen Beschreibung durchgeführt, wobei die genannten Merkmale einzeln oder in beliebiger Kombination vorhanden sein können. Bei dem Verfahren wird vorzugsweise die wenigstens eine Pflanze und/oder wenigstens eine aus mehreren aneinandergereihten Pflanzen ausgebildete Pflanzenreihe, insbesondere mit Hilfe wenigstens einer Positioniereinheit der Agrarvorrichtung, ausgelenkt, aufgefächert und/oder separiert wird. Zusätzlich oder alternativ wird bei dem Verfahren die wenigstens eine Pflanze, insbesondere mit Hilfe wenigstens einer Analyseeinheit, erfasst und/oder analysiert.

Ferner wird ein Computerimplementiertes Verfahren zum Analysieren wenigstens einer Pflanze, insbesondere Getreidepflanze, vorgeschlagen. Bei dem computerimplementierten Verfahren wird die Pflanze vorzugsweise mit Hilfe einer Analyseeinheit erfasst und/oder analysiert. Zusätzlich oder alternativ wird ein durch die Analyseeinheit erfasster Ist-Zustand der Pflanze, beispielsweise die Biomasse, Ertragskennzahl, Krankheiten, Schädlingsbefall und/oder Morphologie, analysiert und/oder kategorisiert. So kann vorzugsweise der Ist-Zustand der wenigstens einen Pflanze mit dem Ist-Zustand wenigstens einer anderen Pflanze und/oder einem Soll-Zustand verglichen werden und/oder die wenigstens eine Pflanze behandelt, markiert und/oder gedüngt werden.

Weitere Vorteile der Erfindung sind in den nachfolgenden Ausführungsbeispielen beschrieben. Es zeigt:
- **Figur 1**: eine schematische Vorderansicht einer Agrarvorrichtung gemäß einem Ausführungsbeispiel, und
- **Figur 2**: eine schematische perspektivische Darstellung einer Agrarvorrichtung gemäß einem alternativen Ausführungsbeispiel und
- **Figur 3**: eine schematische perspektivische Darstellung einer Vereinzelungseinrichtung gemäß einem weiteren Ausführungsbeispiel.

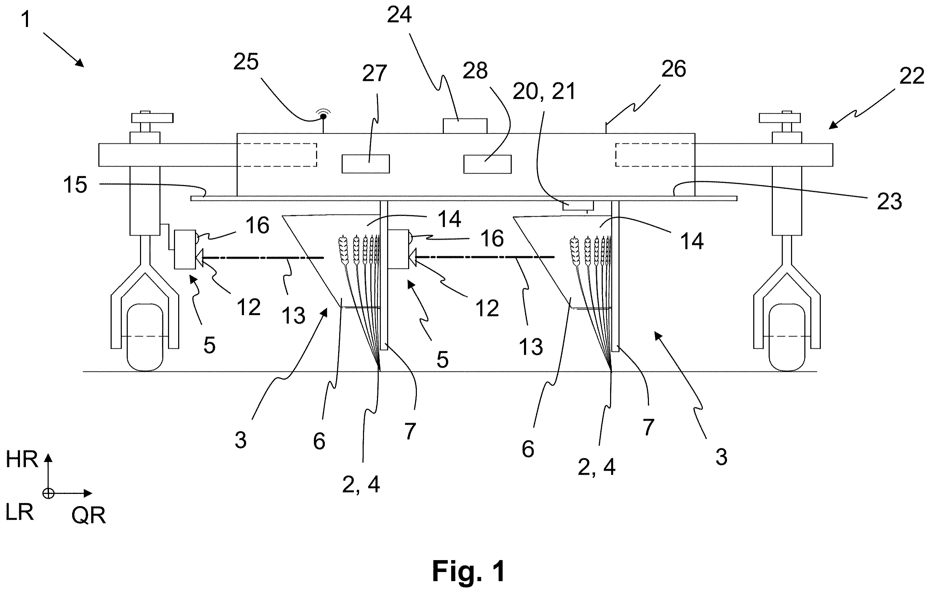

Figur 1 zeigt eine schematische Vorderansicht einer Agrarvorrichtung 1 gemäß einem Ausführungsbeispiel. Die Agrarvorrichtung 1 ist ausgebildet, wenigstens eine Pflanze 2 zu erfassen und/oder zu analysieren. Vorzugsweise ist die Agrarvorrichtung 1 als Boniturvorrichtung ausgebildet, so dass mit dessen Hilfe die wenigstens eine Pflanze 2, insbesondere als Getreidepflanze, eine sogenannte Bonitur durchführbar ist.

Das Ausführungsbeispiel der Figur 1 umfasst wenigstens eine Positioniereinheit 3, die dazu dient, die mindestens eine Pflanze 2 zu positionieren, auszulenken, aufzufächern und/oder zu separieren. Alternativ oder zusätzlich kann die Positioniereinheit 3 dazu verwendet werden, eine aus mehreren aneinandergereihten Pflanzen 2 bestehende Pflanzenreihe 4 zu manipulieren. Des Weiteren beinhaltet das Ausführungsbeispiel der Figur 1 wenigstens eine Analyseeinheit 5, die das Erfassen und/oder Analysieren der mindestens einen Pflanze 2 ermöglicht. Wie dargestellt, kann die Agrarvorrichtungen 1 mehrere Positioniereinheiten 3 und/oder Analyseeinheiten 5 umfassen. So können beispielsweise mehrere Pflanzenreihen 4 positioniert und/oder erfasst und/oder analysiert werden. Im gezeigten Ausführungsbeispiel sind beispielhaft zwei Pflanzenreihen 4 und somit vorteilhafterweise zwei Positioniereinheiten 3 und zwei Analyseeinheiten 5 dargestellt.

Die Positioniereinheit 3 umfasst mindestens ein Leitelement 6, das für das Führen, Umlenken und/oder Auslenken der mindestens einen Pflanze 2 und/oder der Pflanzenreihe 4 konzipiert ist. Zudem umfasst die Positioniereinheit 3 wenigstens ein Aufnahmeelement 7, welches vorzugsweise für das Halten des mindestens einen Leitelements 6 vorgesehen ist.

Das mindestens eine Leitelement 6 ist so an dem mindestens einen Aufnahmeelement 7 angeordnet, dass es entlang einer Längsrichtung LR und/oder Querrichtung QR der Agrarvorrichtung 1 von dem mindestens einen Aufnahmeelement 7 absteht.

Die Analyseeinheit 5 der in Figur 1 dargestellten Agrarvorrichtung 1 weist mindestens einen Bildsensor 12 auf. Dieser Bildsensor 12 dient dem Zweck, einen Ist-Zustand der mindestens einen Pflanze 2 zu erfassen. Der Ist-Zustand umfasst beispielweise die Biomasse, Ertragskennzahl, Krankheiten, Schädlingsbefall und/oder Morphologie der Pflanze.

Die Agrarvorrichtung 1 ist vorzugsweise derart gestaltet, dass dem mindestens einen Leitelement 6 mindestens eine Analyseeinheit 5 zugeordnet ist. Es ist von Vorteil, wenn das mindestens eine Leitelement 6 und die mindestens eine zugeordnete Analyseeinheit 5, insbesondere entlang einer Erfassungsachse 13 des Bildsensors 12 der Analyseeinheit 5, voneinander beabstandet sind.

Das mindestens eine Leitelement 6, speziell der Umlenkabschnitt 10 desselben, umfasst einen Analyseabschnitt 14, welcher vorzugsweise geschlossen, homogen, einfarbig und/oder einseitig am Leitelement 6 angebracht ist. Es ist förderlich, wenn die Erfassungsachse 13 des Bildsensors 12 im Bereich des Analyseabschnitts 14 auf das mindestens eine Leitelement 6 auftrifft. Im bestimmungsgemäßen Gebrauch der Agrarvorrichtung 1 gleitet die Pflanze 2 am Leitelement 6 vorbei, so dass diese zumindest kurzzeitig im Bereich der Erfassungsachse 13 angeordnet ist und so vom Bildsensor 12 erfasst werden kann.

Die Agrarvorrichtung 1 umfasst im gezeigten Ausführungsbeispiel zusätzlich mindestens eine optionale Abschirmeinrichtung 15, die der Abschirmung der Pflanzen 2 vor natürlichem Licht dient, und mindestens eine optionale Lichtquelle 16, die für die Erzeugung von künstlichem Licht konzipiert ist. Es ist von Nutzen, wenn die Lichtquelle 16 als Blitzlicht gestaltet und/oder gesteuert wird, sodass die Pflanze 2 und/oder der Analyseabschnitt 14 nur kurzzeitig mit hoher Leuchtintensität beleuchtet werden kann.

Die Agrarvorrichtung 1, wie in Figur 1 gezeigt, umfasst weiterhin mindestens eine Behandlungseinheit 20. Diese Einheit ist für das Düngen und Behandeln der mindestens einen Pflanze 2 vorgesehen. Zusätzlich oder alternativ beinhaltet die Agrarvorrichtung 1 auch eine Markierungseinheit 21. Diese dient dem Zweck, die mindestens eine Pflanze 2 zu markieren.

Es ist zusätzlich oder alternativ von Vorteil, wenn die Agrarvorrichtung 1 eine Bewegungseinheit 22 aufweist. Diese ermöglicht das Bewegen der Agrarvorrichtung 1 beispielsweise entlang der Längsrichtung LR der Agrarvorrichtung 1, entlang der mindestens einen Pflanze 2 und/oder entlang der sich entlang der Längsrichtung LR erstreckenden Pflanzenreihe 4. Ebenso kann die Agrarvorrichtung 1 mit Hilfe der Bewegungseinheit 22 entlang der Querrichtung QR und/oder Längsrichtung LR und/oder Hochrichtung HR bewegt werden.

Die Positioniereinheit 3 der in Figur 1 dargestellten Agrarvorrichtung 1 umfasst mindestens einen Befestigungsabschnitt 23. Mittels dieses Befestigungsabschnitts 23 ist es möglich, die Positioniereinheit 3, die Analyseeinheit 5, die Behandlungseinheit 20 und/oder die Vereinzelungseinrichtung 17, insbesondere verschiebefest, an der Bewegungseinheit 22 der Agrarvorrichtung 1 zu befestigen.

Die Agrarvorrichtung 1, gemäß Figur 1, beinhaltet außerdem mindestens eine Sensoreinheit 24. Diese dient dem Erfassen von Umgebungsdaten. Zudem umfasst die Agrarvorrichtung 1 eine Funkeinheit 25, die zum Empfangen und/oder Versenden von Daten konzipiert ist. Darüber hinaus beinhaltet die Agrarvorrichtung 1 eine Ortungseinheit 26, welche die Aufgabe hat, die aktuelle Position der Agrarvorrichtung 1 zu bestimmen. Es ist jedoch vorstellbar, dass die Agrarvorrichtung 1 lediglich die eine Sensoreinheit 24, die eine Funkeinheit 25 und/oder die eine Ortungseinheit 26 aufweist.

Es ist ebenfalls von Vorteil, wenn die Agrarvorrichtung 1 mindestens eine Recheneinheit 27 aufweist. Diese ist dafür vorgesehen, die Agrarvorrichtung 1 zu steuern und/oder zu regeln, insbesondere die Positioniereinheit 3, die Analyseeinheit 5, die Behandlungseinheit 20, die Vereinzelungseinrichtung 17, die Bewegungseinheit 22, die Sensoreinheit 24, die Funkeinheit 25 und/oder die Ortungseinheit 26.

Die in Figur 1 dargestellte Agrarvorrichtung 1 umfasst zudem mindestens eine Speichereinheit 28. Diese dient dem Speichern des durch die Analyseeinheit 5 erfassten Ist-Zustands und/oder eines Soll-Zustands der Pflanze 2.

Im gezeigten Ausführungsbeispiel umfasst die Recheneinheit 27 ein Computerprogramm, das vorzugsweise künstliche Intelligenz nutzt und/oder mittels maschinellen Lernens weiterentwickelt wird. Dadurch kann der durch die Analyseeinheit 5 erfasste Ist-Zustand der Pflanze 2 analysiert und/oder kategorisiert werden. Insbesondere kann so der Ist-Zustand der mindestens einen Pflanze 2 mit dem Ist-Zustand mindestens einer anderen Pflanze 2 und/oder einem Soll-Zustand verglichen werden. Weiterhin kann dies dazu dienen, die mindestens eine Pflanze 2 zu behandeln, zu markieren und/oder zu düngen.

Zum vorliegenden Ausführungsbeispiel der Figur 1 sind selbstverständlich abweichende Ausgestaltungen der Erfindung möglich. So ist es beispielsweise vorstellbar dass die mehreren Leitelemente 6 die wenigstens eine Pflanzenreihe 4 und/oder mehrere Pflanzenreihen 4 derart führen, umlenken und/oder auslenken, dass zwischen zwei Leitelementen 6 eine der Pflanzenreihe 4, insbesondere als freistehende Pflanzenreihe, angeordnet ist. So könnte beispielsweise das rechte in Figur 1 gezeigte Leitelemente 6 umgedreht werden, so dass die rechte Pflanzenreihe 4 nach außen geführt, gelenkt und/oder ausgelenkt wird. Zwischen den bereits gezeigten Pflanzenreihen 4 könnte dabei die wenigstens eine freistehende Pflanzenreihe angeordnet sein. Diese freistehende Pflanzenreihe kann im Anschluss mit Hilfe der wenigstens einen Analyseeinheit 5 und/oder mehreren Analyseeinheiten 5 erfasst und/oder analysiert werden. So ist es denkbar, dass die eine oder die mehreren Analyseeinheiten 5 den einen oder mehrere der Bildsensoren12 und/oder Sensoren umfassen. So ist eine mehrdimensionale Erfassung und/oder Analyse realisierbar.

Die eine oder die mehreren Analyseeinheiten 5 und/oder der eine oder mehrere der Bildsensoren12 und/oder Sensoren können beispielsweise, wie dargestellt, an der wenigstens einen Positioniereinheit 3 angeordnet sein. Zusätzlich oder alternativ können die eine oder die mehreren Analyseeinheiten 5 die eine oder die mehreren Analyseeinheiten 5 und/oder der eine oder mehrere der Bildsensoren12 und/oder Sensoren an der Bewegungseinheit 22, am Befestigungsabschnitt 23 und/oder an einer Plattform der Agrarvorrichtung 1 angeordnet sein. Auch bewegbar ausgebildete Analyseeinheiten 5 und/oder Bildsensoren 12 und/oder Sensoren sind vorstellbar.

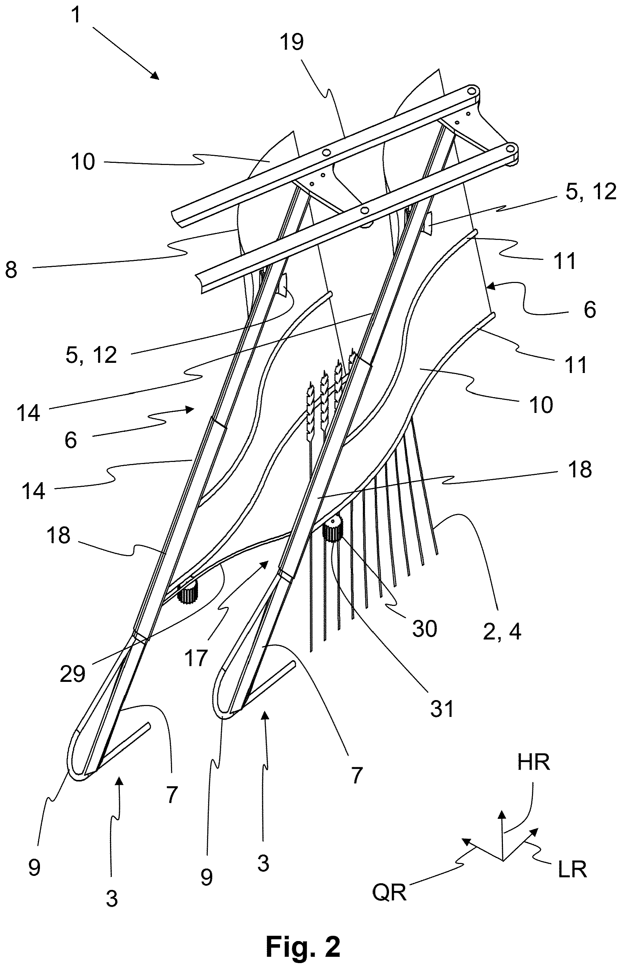

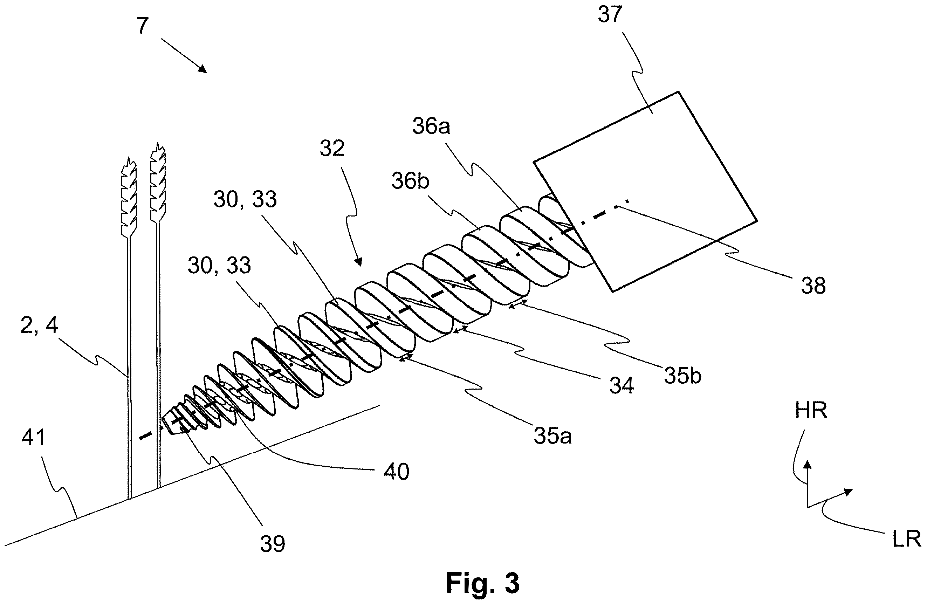

Bei der nachfolgenden Beschreibung des in der Figur 2 dargestellten alternativen Ausführungsbeispiels und/oder des in der Figur 3 dargestellten weiteren Ausführungsbeispiels werden für Merkmale, die im Vergleich zum in Figur 1 dargestellten ersten Ausführungsbeispiel in ihrer Ausgestaltung und/oder Wirkweise identisch und/oder zumindest vergleichbar sind, gleiche Bezugszeichen verwendet. Sofern diese nicht nochmals detailliert erläutert werden, entspricht deren Ausgestaltung und/oder Wirkweise der Ausgestaltung und Wirkweise der vorstehend bereits beschriebenen Merkmale.

Die Figur 2 zeigt eine schematische perspektivische Darstellung einer Agrarvorrichtung 1 gemäß einem alternativen Ausführungsbeispiel. Hierin sind im Vergleich zum Ausführungsbeispiel der Figur 1 primär mehr Details der Positioniereinheit 3 und einer Vereinzelungseinrichtung 17 erkennbar. Auf die Darstellung der Erfassungsachse 13, des Analyseabschnitts 14, der Abschirmeinheit 15, der Lichtquelle 16, der Behandlungseinheit 20, der Markierungseinheit 21, der Bewegungseinheit 22, des Befestigungsabschnitts 23, der Sensoreinheit 24, der Funkeinheit 25, der Ortungseinheit 26, der Recheneinheit 27 und der Speichereinheit 28 wurde aus Übersichtsgründen verzichtet. Die Positioniereinheit 3 und/oder die Analyseeinheit 5 und/oder die Vereinzelungseinrichtung 17 der Figur 2 kann die vorstehend genannten Merkmale ebenso aufweisen.

Die Darstellung der Agrarvorrichtung 1 in Figur 2 zeigt ein Leitelement 6, das zumindest abschnittsweise schräg zur Längsrichtung LR und/oder Querrichtung QR verläuft und mindestens eine Biegung 8 umfasst. In der Draufsicht der Agrarvorrichtung 1 ist das mindestens eine Leitelement 6, insbesondere mit zwei Biegungen 8, S-förmig ausgebildet. Weiterhin weist das Leitelement 6 einen Führungsabschnitt 9 auf. Der Führungsabschnitt 9 Abschnitt dient dazu, die mindestens eine Pflanze 2, im bestimmungsgemäßen Gebrauch der Agrarvorrichtung 1, besonders im Bereich eines Halms der Pflanze, zu leiten und/oder auszulenken.

Das Leitelement 6 beinhaltet zudem einen Umlenkabschnitt 10, der für die Führung und/oder Auslenkung der Pflanze 2 konzipiert ist, insbesondere im Bereich wenigstens einer Frucht, beispielsweise eines Korns und/oder einer Ähre, und/oder eines Blatts der Pflanze. Die Pflanze 2 wird vorzugsweise mittels des Führungsabschnitts 9 zum Umlenkabschnitt 10 geleitet und mittels des Umlenkabschnitts 10 entlang der Querrichtung QR ausgelenkt. Im Bereich des Umlenkabschnitts 10 umfasst das Leitelement 6 mindestens eine Stützstruktur 11. Durch diese Stützstruktur 11 wird das Leitelement 6 an dem Aufnahmeelement 7 angeordnet und/oder befestigt und spannt den Umlenkabschnitt 10 auf.

Die Agrarvorrichtung 1 gemäß Figur 2 umfasst die vorstehend bereits erwähnte Vereinzelungseinrichtung 17, die für das Separieren, Aufreihen, Trennen, Stabilisieren und/oder Begrenzen der Bewegung bzw. Auslenkung der Pflanzenreihe 4 in einzelne Pflanzen 2 ausgebildet ist. Diese Vereinzelungseinrichtung 17 weist bevorzugt mindestens ein Trennelement 30 auf, mit dessen Hilfe die Pflanzen 2 voneinander getrennt werden kann. Im gezeigten Ausführungsbeispiel ist das Trennelement 30 als Zahn eines Förderrads 31 ausgebildet. Zusätzlich oder alternativ kann das Trennelement 30 beispielsweise als Bürste, Zahn, Stab, Bolzen und/oder Finger ausgebildet sein.

Ebenfalls ist es vorstellbar, dass das Trennelement 30 an ein Förderband, an einem Riemen, an einer Riemenbürste und/oder an einem Zahnriemen angeordnet ist. Des Weiteren umfasst die Vereinzelungseinrichtung 17 im gezeigten Ausführungsbeispiel ein Andrückelement 29. Mit Hilfe des Andrückelements 29 kann die wenigstens eine Pflanze 2 zum Trennelement 30 und/oder zum Förderrad 31 und/oder zum Riemen und/oder zur Riemenbürste und/oder zum Zahnriemen gedrückt werden. Vorzugsweise ist das Andrückelement 29 als Federelement ausgebildet, so dass das Andrücken elastisch bzw. federnd erfolgt.

Zusätzlich oder alternativ zu den vorstehend genannten Merkmalen der Vereinzelungseinrichtung 17 kann diese eine Düse umfassen, mit dessen Luftstrom die wenigstens eine Pflanze 2 angedrückt und/oder vereinzelt und/oder separiert werden kann. Das Andrückelement 29, zusammen mit einem Federelement und/oder der Düse, kann nach der Vereinzelung für die Stabilisierung der Pflanze 2 sorgen und/oder ihre Bewegung begrenzen, was die nachfolgende Analyse verbessert.

Die Positioniereinheit 3 umfasst mindestens eine Stelleinrichtung 18, 19, wobei eine erste Stelleinrichtung 18 als Linearstelleinrichtung zum Verstellen des Leitelements 6 entlang der Hochrichtung HR und/oder Längsrichtung LR dient. Eine zweite Stelleinrichtung 19 ist entweder als Rotationsstelleinrichtung zum Verdrehen des Leitelements 6 oder als Linearstelleinrichtung zum Verstellen des Leitelements 6 entlang der Querrichtung QR ausgelegt, wodurch Pflanzen 2, Früchte und/oder Blätter von Pflanzenreihen 4 unterschiedlicher Breiten erfasst und/oder analysiert werden können.

In der Agrarvorrichtung 1 sind mehrere Leitelemente 6 und mehrere Analyseeinheiten 5 integriert, wobei mindestens einem der Leitelemente 6 und/oder mehreren der Leitelemente 6 mindestens eine der Analyseeinheiten 5 zugeordnet ist. Die dem Leitelement 6 zugeordnete Analyseeinheit 5 und/oder Vereinzelungseinrichtung 17 ist an einem benachbarten Leitelement 6 der Positioniereinheit 3, insbesondere an der Stützstruktur 11 des benachbarten Leitelements 6, montiert. Dadurch wird gewährleistet, dass bei Verstellung des Leitelements 6 und/oder der Positioniereinheit 3 mittels der Stelleinrichtung 18, 19 auch die Analyseeinheit 5 und/oder Vereinzelungseinrichtung 17 entsprechend mitverstellt wird, was eine flexible Anpassung an verschiedene Pflanzentypen und -zustände ermöglicht.

Die Figur 3 zeigt eine schematische perspektivische Darstellung einer Vereinzelungseinrichtung 17 gemäß einem weiteren Ausführungsbeispiel. Im Gegensatz zur Vereinzelungseinrichtung 17 des Ausführungsbeispiels der Figur 2 umfasst die in Figur 3 dargestellte Vereinzelungseinrichtung 17 eine Schneckenwelle 32. Im Gegensatz zu den vorstehenden Ausführungsbeispielen der Figuren 1 und/oder 2 wurde auf die Darstellung einiger Merkmale der Agrarvorrichtung 1, insbesondere auf die Darstellung vieler Bestandteile der Positioniereinheit 3, der Analyseeinheit 5, das Leitelement 6, der Erfassungsachse 13, des Analyseabschnitts 14, der Abschirmeinrichtung 15, der Lichtquelle 16, der Behandlungseinheit 20, der Markierungseinheit 21, der Bewegungseinheit 22, des Befestigungsabschnitts 23, der Sensoreinheit 24, der Funkeinheit 25, der Ortungseinheit 26, der Recheneinheit 27 und der Speichereinheit 28 aus Übersichtsgründen verzichtet.

Hierbei sei darauf hingewiesen, dass die Vereinzelungseinrichtung 17 mit der Agrarvorrichtung 1 der Figuren 1 und 2 ohne Weiteres kombinierbar ist. Insbesondere ist es denkbar, dass die Vereinzelungseinrichtung 17 gemäß Figur 3 an dem Leitelement 6 und/oder an dem Umlenkabschnitt 10 und/oder an der Stützstruktur 11 der Figur 1 und/oder 2 angeordnet ist.

Die Vereinzelungseinrichtung 17 gemäß Figur 3 umfasst beispielhaft eine Schneckenwelle 32. Die Schneckenwelle 32 umfasst eine innere Welle und wenigstens eine um diese Welle wendelförmig angeordnete Wendel 36. Im gezeigten Ausführungsbeispiel umfasst die Schneckenwelle 32 zwei Wendeln 36a und 36b. Somit kann die Schneckenwelle 32 als Zweigangschneckenwelle bezeichnet werden. Zusätzlich oder alternativ umfasst die wenigstens eine Wendel 36 eine doppelte Steigung. So kann das Vereinzeln der Pflanzen verbessert werden.

Die Vereinzelungseinrichtung 17 umfasst im gezeigten Ausführungsbeispiel wenigstens ein Antriebselement 37. Das Antriebselement 37 ist vorzugsweise ein Antriebsmotor. Zusätzlich oder alternativ kann das Antriebselement 37 auch eine Welle und/oder ein Riementrieb und/oder ein Getriebe sein und/oder umfassen. Mittels des Antriebselements 37 kann die Schneckenwelle 32 um eine Drehachse 38 gedreht werden. Im gezeigten Ausführungsbeispiel ist die Schneckenwelle 32 lediglich am Antriebselement 37 angeordnet und/oder aufgenommen und/oder gelagert. Zusätzlich oder alternativ könnte die Schneckenwelle 32 an einem Anfangsabschnitt 39 der Schneckenwelle 32 drehbar gelagert und/oder angetrieben werden.

Die Schneckenwelle 32 umfasst im gezeigte Ausführungsbeispiel das wenigstens eine Trennelement 30 und/oder wenigstens einen Trennabschnitt 33. Die Bezeichnung Trennabschnitt 33 ist hierbei wohl passender, da bei der vorliegenden Schneckenwelle 32 sogenannte Wendelabschnitte der wenigstens eine Wendel 36 den wenigstens einen Trennabschnitt 33 und/oder das wenigstens eine Trennelement 30 bereitstellen. Nichtsdestotrotz haben das Trennelement 30 (insbesondere gemäß Figur 2) und der Trennabschnitt 33 (insbesondere gemäß Figur 3) die selbe oder zumindest eine ähnliche Wirkung. Mit Hilfe dieser Trennabschnitte 33 und/oder Trennelemente 30 können einzelne Pflanzen 2 der Pflanzenreihe 4 voneinander getrennt und/oder aufgefächert und/oder separiert werden.

So sind die Trennabschnitte 33 jeweils voneinander durch einen Abstand 34 getrennt. Insbesondere sind die Trennabschnitte 33 im gezeigten Ausführungsbeispiel einer der beiden Wendeln 36a und 36b durch zwei der Abstände 34 und eine Wandstärke 35 voneinander getrennt. Dadurch wird deutlich, dass die vorliegende Schneckenwelle 32 eine hohe Steigung umfasst.

Der Anfangsabschnitt 39 der Schneckenwelle 32 ist im gezeigten Ausführungsbeispiel, insbesondere konisch, verjüngt. Dadurch kann die Vereinzelungseinrichtung 17, insbesondere die Schneckenwelle 32 vereinfacht zwischen zwei Pflanzenzeihen 4 eingebracht werden. Im Bereich des konisch verjüngten Anfangsabschnitts 39 sind vorzugsweise zudem die Abstände 34, die Wandstärken 35 und/oder die Wendelhöhen sehr gering. So kann die Pflanze 2 vereinfacht, insbesondere im Bereich einer Wurzel, in den wenigstens einen Abstand 34 eingebracht werden. Dabei beträgt die Distanz des Anfangsabschnitts 39 der Schneckenwelle 32 zum Untergrund 41 vorzugsweise weniger als 10 cm, beispielsweise weniger als 5 cm.

Wird die Pflanze 2 durch die Schneckenwelle 32 aufgegriffen, so wird sie durch Drehung der Schneckenwelle 32 um die Drehachse 38 entlang der Längsrichtung LR und/oder Hochrichtung HR transportiert. Da sich die Drehachse 38 entlang der Längsrichtung LR erstreckt, sollte die Drehung um die Drehachse 38 mit der Bewegung der Bewegungseinheit 22 der Agrarvorrichtung 1 (siehe Figur 1) synchronisiert werden. Dadurch bleibt die Position der Pflanze 2 im bestimmungsgemäßen Gebrauch der Vereinzelungseinrichtung 17 im Wesentlichen konstant bzw. wird die Frucht lediglich leicht ausgelenkt. So kann die Kraft auf die Wurzel und/oder den Halm der Pflanze 2 reduziert werden. Die im Ausführungsbeispiel der Figur 3 gezeigten verändernden Wandstärken 35a und 35b der Wendelabschnitte und/oder Trennabschnitte 33 bewirken dabei, dass die Pflanzen 2 aufgefächert und/oder im Bereich der Frucht separiert werden. Das Auffächern und/oder Separieren der Pflanzen 2 wird bei zunehmender Bewegung mehr, so dass die Analyse der einzelnen Pflanze 2 und/oder deren Frucht vereinfacht und/oder verbessert wird. Zusätzlich oder alternativ zum gezeigten Beispiel kann sich der Abstand 34 der Trennabschnitte 33 entlang der Längsrichtung LR, der Hochrichtung HR und/oder der Drehachse 38 verändern.

Des Weiteren zeigt die Vereinzelungseinrichtung 17 der Figur 3 eine Oszillationseinrichtung 40, mit dessen Hilfe eine oszillierende Bewegung in die Pflanze 2 eingebracht werden kann. Im gezeigten Ausführungsbeispiel ist die Oszillationseinrichtung 40 als eine oder mehrere Erhöhungen an der Kernwelle der Schneckenwelle 32 ausgebildet. Durch diese Erhöhungen kann die Pflanze 2 und/oder die Schneckenwelle 32 oszillierend bewegt werden. Zusätzlich oder alternativ kann die Schneckenwelle 32 mit geeigneten Mitteln, beispielsweise mittels einem Stellmotor und/oder einer Hydraulik und/oder eine Pneumatik, oszillierend bewegt werden.

In Figur 3 ist dargestellt, dass die Schneckenwelle 32 auf die wenigstens eine Pflanze 2 einwirkt. Um die wenigstens eine Pflanze 2 sicher in dem wenigstens einem Abstand 34 zu halten, kann die Vereinzelungseinrichtung 17 wenigstens eine zweite Schneckenwelle 32 und/oder ein Förderband und/oder einen Riemen und/oder eine Riemenbürste und/oder einen Zahnriemen und/oder ein Andrückelement 29 (siehe Figur 2) und/oder ein Federelement umfassen. Ebenfalls ist es vorstellbar, dass die Wirkung der hier dargestellten Schneckenwelle 32 wenigstens ein Förderband und/oder einen Riemen und/oder eine Riemenbürste und/oder einen Zahnriemen und/oder ein Förderrad 31 (siehe Figur 2) und/oder mehrere der Förderräder 31 (siehe Figur 2) und/oder ein Andrückelement 29 (siehe Figur 2) und/oder ein Federelement und/oder eine Düse und/oder ein Förderelement und/oder eine Spindel und/oder eine Spirale übernimmt/übernehmen.

### Bezugszeichenliste

- 1: Agrarvorrichtung
- 2: Pflanze
- 3: Positioniereinheit
- 4: Pflanzenreihe
- 5: Analyseeinheit
- 6: Leitelement
- 7: Aufnahmeelement
- 8: Biegung
- 9: Führungsabschnitt
- 10: Umlenkabschnitt
- 11: Stützstruktur
- 12: Bildsensor
- 13: Erfassungsachse
- 14: Analyseabschnitt
- 15: Abschirmeinrichtung
- 16: Lichtquelle
- 17: Vereinzelungseinrichtung
- 18: erste Stelleinrichtung
- 19: zweite Stelleinrichtung
- 20: Behandlungseinheit
- 21: Markierungseinheit
- 22: Bewegungseinheit
- 23: Befestigungsabschnitt
- 24: Sensoreinheit
- 25: Funkeinheit
- 26: Ortungseinheit
- 27: Recheneinheit
- 28: Speichereinheit
- 29: Andrückelement
- 30: Trennelement
- 31: Förderrad
- 32: Schneckenwelle
- 33: Trennabschnitt
- 34: Abstand
- 35: Wandstärke
- 36: Wendel
- 37: Antriebselement
- 38: Drehachse
- 39: Anfangsabschnitt
- 40: Oszillationseinrichtung
- 41: Untergrund

- LR: Längsrichtung
- QR: Querrichtung
- HR: Hochrichtung

## Patentansprüche

1. Agrarvorrichtung (1) zum Erfassen und/oder Analysieren wenigstens einer Pflanze (2), insbesondere Getreidepflanze,
mit wenigstens einer Positioniereinheit (3) zum Positionieren, Auslenken, Auffächern und/oder Separieren der wenigstens einen Pflanze (2) und/oder wenigstens einer aus mehreren aneinandergereihten Pflanzen (2) ausgebildeten Pflanzenreihe (4), und
mit wenigstens einer Analyseeinheit (5) zum Erfassen und/oder Analysieren der wenigstens einen Pflanze (2).

2. Agrarvorrichtung nach dem vorangegangenen Anspruch, **dadurch gekennzeichnet, dass** die Positioniereinheit (3) wenigstens ein Leitelement (6) zum Führen, Umlenken und/oder Auslenken der wenigstens einen Pflanze (2) und/oder der wenigstens einen Pflanzenreihe (4) und/oder
wenigstens ein, insbesondere stangenförmiges, Aufnahmeelement (7) für das wenigstens eine Leitelement (6) umfasst.

3. Agrarvorrichtung nach einem der vorangegangenen Ansprüche, **dadurch gekennzeichnet, dass** das wenigstens eine Leitelement (6) an dem wenigstens einen Aufnahmeelement (7) angeordnet ist und/oder entlang einer Längsrichtung (LR) und/oder Querrichtung (QR) der Agrarvorrichtung (1) von dem wenigstens einen Aufnahmeelement (7) absteht.

4. Agrarvorrichtung nach einem der vorangegangenen Ansprüche, **dadurch gekennzeichnet, dass** das wenigstens eine Leitelement (6) einen Führungsabschnitt (9) und/oder einen Umlenkabschnitt (10) umfasst, wobei sich der Führungsabschnitt (9) vorzugsweise zumindest abschnittsweise entlang der Längsrichtung (LR) erstreckt und/oder der Umlenkabschnitt (10) vorzugsweise zu der Querrichtung (QR) und/oder der Längsrichtung (LR) schräg verläuft und/oder eine Biegung (8) umfasst.

5. Agrarvorrichtung nach einem der vorangegangenen Ansprüche, **dadurch gekennzeichnet, dass** die wenigstens eine Analyseeinheit (5) wenigstens einen Bildsensor (12) zum Erfassen eines Ist-Zustands der wenigstens einen Pflanze (2), beispielsweise der Biomasse, Ertragskennzahl, Krankheiten, Schädlingsbefall und/oder Morphologie, umfasst,
wobei der Bildsensor (12) vorzugsweise als RGB-Bildsensor, NIR-Bildsensor, SWIR-Bildsensor, MSI-Bildsensor und/oder HSI-Bildsensor ausgebildet ist.

6. Agrarvorrichtung nach einem der vorangegangenen Ansprüche, **dadurch gekennzeichnet, dass** dem wenigstens einen Leitelement (6) die wenigstens eine Analyseeinheit (5) zugeordnet ist,
wobei vorzugsweise das wenigstens eine Leitelement (6) und die wenigstens eine zugeordnete Analyseeinheit (5), insbesondere entlang einer Erfassungsachse (13) des Bildsensors der Analyseeinheit (5), voneinander beabstandet sind.

7. Agrarvorrichtung nach einem der vorangegangenen Ansprüche, **dadurch gekennzeichnet, dass** das wenigstens eine Leitelement (6), insbesondere der Umlenkabschnitt (10) des Leitelements (6), einen, insbesondere geschlossenen, homogenen, einfarbigen und/oder einseitig am Leitelement (6) angeordneten, Analyseabschnitt (14) umfasst,
wobei die Erfassungsachse (13) des Bildsensors (12) im Bereich des Analyseabschnitts (14) auf das wenigstens eine Leitelement (6) auftrifft und/oder die wenigstens eine Pflanze (2), vorzugsweise die Frucht und/oder das Blatt, der wenigstens einen Pflanze (2), im bestimmungsgemäßen Gebrauch der Agrarvorrichtung (1) entlang der Erfassungsachse (13) zwischen dem Analyseabschnitt (14) des Leitelements (6) und dem Bildsensor (12) der Analyseeinheit (5) angeordnet ist.

8. Agrarvorrichtung nach einem der vorangegangenen Ansprüche, **dadurch gekennzeichnet, dass** die Agrarvorrichtung (1) wenigstens eine Vereinzelungseinrichtung (17) zum Separieren, Aufreihen, Trennen, Stabilisieren und/oder Begrenzen der Pflanzenreihe (4) in einzelne Pflanzen (2) umfasst,
wobei die Vereinzelungseinrichtung (17) vorzugsweise wenigstens ein Trennelement (30) und/oder ein Förderband und/oder einen Riemen und/oder eine Riemenbürste und/oder einen Zahnriemen und/oder ein Förderrad (31) und/oder ein Andrückelement (29) und/oder ein Federelement und/oder eine Düse und/oder eine **Schneckenwelle** (32) und/oder wenigstens einen **Trennabschnitt** (33) aufweist.

9. Agrarvorrichtung nach einem der vorangegangenen Ansprüche, **dadurch gekennzeichnet, dass** Förderband und/oder der Riemen und/oder die Riemenbürste und/oder der Zahnriemen und/oder die wenigstens eine Schneckenwelle (32) und/oder das wenigstens eine Förderrad (31) mehrere entlang der Längsrichtung (LR) und/oder entlang einer Hochrichtung (HR) der Agrarvorrichtung (1) und/oder entlang einer **Drehachse** (38) voneinander beabstandete Trennelemente (30) und/oder Trennabschnitte (33) umfasst,
wobei sich ein **Abstand** (34) zwischen den mehreren Trennelementen (30) und/oder Trennabschnitte (33) und/oder eine **Wandstärke** (35) der mehreren Trennelemente (30) und/oder Trennabschnitte (33) entlang der Längsrichtung (LR) und/oder der Hochrichtung (HR) und/oder der Drehachse (38) zumindest abschnittsweise verändert.

10. Agrarvorrichtung nach einem der vorangegangenen Ansprüche, **dadurch gekennzeichnet, dass** die wenigstens eine Schneckenwelle (32) wenigstens eine **Wendel** (36), insbesondere wenigstens zwei Wendeln (36), umfasst und/oder sich zu einem **Anfangsabschnitt** (39), insbesondere konisch, verjüngt,
wobei die Wendel (36) vorzugsweise das wenigstens eine Trennelement (30) und/oder den wenigstens einen Trennabschnitt (33) bereitstellt.

11. Agrarvorrichtung nach einem der vorangegangenen Ansprüche, **dadurch gekennzeichnet, dass** die Vereinzelungseinrichtung (17) wenigstens ein **Antriebselement** (37) zum Antreiben des Förderbands und/oder des Riemens und/oder der Riemenbürste und/oder des Zahnriemens und/oder der Schneckenwelle (32) und/oder des Förderrads (31) und/oder wenigstens eine **Oszillationseinrichtung** (40) zum Einbringen einer oszillierenden Bewegung in die Pflanze (2) umfasst.

12. Agrarvorrichtung nach einem der vorangegangenen Ansprüche, **dadurch gekennzeichnet, dass** die Agrarvorrichtung (1) eine Recheneinheit (27) zum Steuern und/oder Regeln der Agrarvorrichtung (1), insbesondere der Positioniereinheit (3), der Analyseeinheit (5), einer Behandlungseinheit (20), der Vereinzelungseinrichtung (17), einer Bewegungseinheit (22), einer Sensoreinheit (24), einer Funkeinheit (25), einer Ortungseinheit (26), des Antriebselements (37) und/oder der Oszillationseinrichtung (40) umfasst.

13. Vereinzelungseinrichtung (17) für eine Agrarvorrichtung (1), vorzugsweise gemäß einem oder mehreren der vorherigen Ansprüche, **dadurch gekennzeichnet,**
**dass** die Vereinzelungseinrichtung (17) zumindest ein in den vorherigen Ansprüchen genanntes und die Vereinzelungseinrichtung (17) betreffendes Merkmal aufweist.

14. Verfahren zum Erfassen und/oder Analysieren wenigstens einer Pflanze (2), insbesondere Getreidepflanze, mittels einer Agrarvorrichtung (1), insbesondere gemäß einem oder mehreren der vorherigen Ansprüche,
wobei die wenigstens eine Pflanze (2) und/oder wenigstens eine aus mehreren aneinandergereihten Pflanzen (2) ausgebildete Pflanzenreihe (4), insbesondere mit Hilfe wenigstens einer Positioniereinheit (3) der Agrarvorrichtung (1), ausgelenkt, aufgefächert und/oder separiert wird, und
wobei die wenigstens eine Pflanze (2), insbesondere mit Hilfe wenigstens einer Analyseeinheit (5), erfasst und/oder analysiert wird.

15. Computerimplementiertes Verfahren zum Analysieren wenigstens einer Pflanze (2), insbesondere Getreidepflanze,
bei dem die Pflanze (2) mit Hilfe einer Analyseeinheit (5) erfasst und/oder analysiert wird,
wobei ein durch die Analyseeinheit (5) erfasster Ist-Zustand der Pflanze (2), beispielsweise die Biomasse, Ertragskennzahl, Krankheiten, Schädlingsbefall und/oder Morphologie, analysiert und/oder kategorisiert wird,
insbesondere um den Ist-Zustand der wenigstens einen Pflanze (2) mit dem Ist-Zustand wenigstens einer anderen Pflanze (2) und/oder einem Soll-Zustand zu vergleichen und/oder um die wenigstens eine Pflanze (2) zu behandeln, zu markieren und/oder zu düngen.
